# EUROPEAN PATENT APPLICATION

(11) **EP 4 678 227 A1**
(43) Date of publication of application: **14.01.2026**
(21) Application number: 24188414.7
(22) Date of filing: 12.07.2024
(51) Int. Cl.: A61N 5/10

(54) **A METHOD FOR OPTIMIZING A RADIATION TREATMENT PLAN**

(71) Applicant: RaySearch Laboratories AB, 104 30 Stockholm (SE)
(72) Inventor: TRANEUS, Erik, 752 39 Uppsala (SE)

(57) **Abstract**

The present disclosure relates to a method for optimizing an existing intensity modulated particle therapy (IMPT) radiation treatment plan for a target in a patient. The target is represented by voxels and the method involves sampling a large number of ions from a radiation source. A three-dimensional object having at least one spike, measuring at least 3mm in length, is placed into the path of the ions. Each ion is traced along a trace through the three-dimensional object and the patient to a voxel, thereby calculating a water equivalent path length (WEPL) value for the voxel and the ion. The WEPL value is then inserted into a WEPL histogram. These steps are repeated for the large number of ions and traces for each voxel representing the target. A dose for a single energy layer treatment plan is calculated using a pencil beam dose engine by looping over the WEPL histogram per voxel until all values in the WEPL histogram for the voxel are accounted for. This step is repeated for each voxel representing the target and the healthy tissues.

## Description

### Technical field

The technical field of the disclosure relates to the treatment of tumors and therewith targets using radiation. In particular the disclosure relates to the optimization of treatment plans for radiation treatment. In a further aspect the disclosure relates to treatment of targets using heavier ions such as oxygen (O) ions, carbon (C) ions, helium (He) ions, neon (Ne) ions and lithium (Li) ions, the suggested method does however also work for protons.

### Technical background

It is known how to optimize ion treatment plans for treatments in a body of a patient. Typical criteria to optimize treatments are reducing treatment time, optimizing for optimal dose delivery in the target and while minimizing dose to the surrounding or exposed non-target tissue area (ENTA) and reducing dose delivery to non-target tissue in general.

In some cases, the quality of a radiotherapy treatment plan is evaluated, to ensure that it will be delivered correctly and affect the patient in the desired way. The delivery of radiation using a radiotherapy treatment plan to a patient is affected by a number of factors, one being the patient's own anatomy. The tissue in the patient's body will lead to scattering of particles, altering their path and therefore affecting the resulting dose distribution in the patient's body and the target. The density of the structures traversed by the particle or ion will affect the path length.

In ion-based radiotherapy each ion will emit most of its energy towards the end of its path, creating what is known as the Bragg peak, which will be explained later herein.

### Summary of this disclosure

Both the geometrical path length and the water equivalent path length (WEPL) are of interest in dose planning. The WEPL is defined as the distance that is equivalent to that measured in water, taking into account the densities of the tissues traversed by the ion and the variation of stopping power ratio between water and the traversed media. It is usually calculated as the product of the distance in the considered materials times the density times the water to medium stopping power ratio. Several methods for determining the water equivalent path length are known. Some dose engines, for example, the Monte Carlo dose engine, have functions for calculating the WEPL as a part of dose planning. Both the geometrical path length and the WEPL may be affected by setup errors. The geometrical path length may be affected in particular where the part of the patient's body that has an irregular shape, such as for example an ear. The WEPL will be affected both by irregular shape and by variations in tissue density. The path length may be determined along a straight line from the entry point to the voxel concerned. More realistically, however, the path will not be a straight line. An alternative way of determining the path length, therefore, is based on the average path length value of all simulated trajectories passing through the voxel. This is valid both for the geometrical path length and for the WEPL.

There is always an amount of uncertainty in dose planning, since factors such as CT calibration, tissue inhomogeneity, organ motion and deformation cannot be controlled. Because of such uncertainty factors, there is a desire for a plan to be as robust as possible, meaning that it should provide the same dose distribution even if some factor changes. It is also important to evaluate the quality of a radiotherapy treatment plan, to ensure that it will be delivered correctly and affect the patient and in particular the target, in the desired way. When evaluating a radiotherapy treatment plan, its robustness is of interest. The robustness reflects how well the plan will work in the case of small changes to the setup. For example, if the patient receiving the treatment moves relative to the assumed position this will affect where the particles will stop and thereby also the treatment. If the tissue type traversed changes as a result of the movement, the effect on the path length may be significant. Therefore, plans involving areas with high tissue inhomogeneity will generally be less robust than plans involving areas with the same or similar tissue type. A plan is said to be robust if a change in any of the above factors will not change the dose. There is a constant desire to provide treatment plans that are as robust as possible, and also to evaluate the robustness of calculated treatment plans.

Generally, the treatment takes place by distributing or shooting ions towards the target and selecting the energy of the ions so they stop at a desired depth in the patient. In a treatment plan the energy of ions are specified by a set of so-called energy layers where each energy layer corresponds to one nominal ion energy. Each energy layer or level that is used to shoot the ion towards the target has a distinct depth of the Bragg peak. If the energy layer is changed, the position of the Bragg peak will be changed. However, changing energy layers during treatment is not desirable as it substantially prolongs the treatment, as further explained below. Another option to modify depth of the Bragg peak is to insert an object, typically a three-dimensional (3D) object into the path of the ions. This way the position of the Bragg peak can be changed within the tissue and therewith within the target, which may result in better treatment. The three-dimensional object must have a specially designed shape in order to manipulate the dose delivery so that the energy of each ion is delivered within the target. The 3D object is thereby used to inflict the required or specified energy modulation.

The goal of the method used for the approach as described above with ions, such as for example proton ions or other ions, is to establish a final and optimized treatment plan. In order to do this, a two-step approach has been implemented and demonstrated starting with the generation of a multi-energy layer intensity modulated therapy plan (IMPT) using the Monte Carlo approach. This first generated plan is herein called a mother plan, initial plan, or primary plan. In a second step the 3D object is generated from an energy fluence fingerprint of the primary plan. The 3D geometry is then applied or used in a single energy layer pencil beam scanning plan, meaning that in the end only one energy layer is needed for the entire plan, which reduces treatment time substantially.

The above-described method using a primary plan and an energy fluence fingerprint or map was initially conceived to work with any ion not only proton ions. The EP 3 957 361 describes a solution to optimize a treatment plan using a 3D object. However, EP 3 957 361 A1 does, among other things, not disclose the use of a Pencil Beam dose engine for a single energy treatment plan and is limited to a Monte Carlo method and dose engine.

Another document that describes radiation methods is Simeonov et al (XP 20319397). Simeonov describes the use of raytracing to discover the depth at which the particle (proton) travels and releases its energy in matter and then calculate the length of a spike or material in order to manipulate the depth and which the particle releases its energy so that the dose is delivered within the target. However, Simeonov et al does not describe to establish a multienergy layer mother plan prior to designing the spikes.

Using heavier ions, in particular C (e.g. carbon) ions is desirable since these ions have a distinct penumbra, meaning the shape of their dose delivery is sharper than with protons, which basically in itself means that the dose delivery with heavier ions is more exact and precise.

Further, heavier ions have a higher toxicity to the target and are therefore more efficient. The Monte Carlo method is impractical to compute dose when used with heavier ions, since calculating the path for heavier ions would take a very long time. In clinical practice, use of the Monte Carlo dose engine is limited to protons which gives a reasonable calculation time. The disclosure aims to provide a new method for treatment planning optimization, a method that works for any ion treatment and significantly reduces delivery time, in particular for heavier ions such as any of helium, lithium, beryllium, boron, carbon, nitrogen, oxygen, fluorine or neon.

It is an object of the present disclosure to provide a method for optimizing an existing intensity modulated particle therapy (IMPT) radiation treatment plan for a target in a patient, which improves the accuracy and efficiency of the treatment planning process.

It is a further aspect of this disclosure to provide a method of optimizing a radiation therapy treatment plan for heavier ions such as helium (He), lithium (Li), beryllium (Be), boron (B), carbon (C), nitrogen (N), oxygen (O), fluorine (F) or neon (Ne) ions.

In still another aspect of this disclosure an aim is to create a single energy layer plan with the same or at least a very similar dose distribution as the IMPT radiation treatment plan that was used as a starting point, whereby such a single energy layer plan is designed to work with any type of ion.

In a pencil beam dose engine, the dose in a voxel is computed or calculated based on the water equivalent path length (WEPL) along a line between the source and the voxel. The WEPL, per voxel, is found by a ray trace between source and voxel that accumulates WEPL increments (WEPL = track length through a tissue type multiplied with density) over the traversed voxels along the ray trace or ray. Thus, for calculating WEPL voxels are used along the entire ray trace along which the ion is traveling. The WEPL per voxel in the target depends on patient anatomy as the patient is not homogeneous.

If an object is inserted in the beam upstream of the patient the objects thickness along the specific ray adds to the WEPL, as the object adds resistance to the ion along the traveled path or ray. An object that can be used is a range shifter. A range shifter is a slab that is infinite as seen along the ray, having a uniform thickness, for example a Plexiglas-disc, placed in the beam to absorb energy before the beam reaches the patient and therewith the target. In a pencil beam does engine, a range shifter can be considered as having a constant thickness across the beam, with a minor adjustment needed as the ions may hit the range shifter at a small angle depending on the distance to the beam axis. If a more complicated object like a three-dimensional object with spikes and with geometry details of size 0.1 mm and greater is inserted into the beam, as suggested in this disclosure, it gets more complicated as such a three-dimensional object can be considered a beam modifier object.

A typical dose voxel is a 2mm x 2mm x 2mm cube. Ions reaching the voxel will have experienced a distribution of path lengths when traveling through the three-dimensional object on their way to the dose voxel. For the patient part of the ray trace there will also be a WEPL spread but this is much smaller compared to the WEPL spread in the three-dimensional object, so the patient part can be disregarded for the calculation. The pin-geometry or spike-geometry of the three-dimensional object has bigger influence on the WEPL spread.

The present disclosure relates to a method to incorporate WEPL spread, whereby WEPL spread is basically depth manipulation of the Bragg peak due to the three-dimensional object when the dose engine is a pencil beam dose engine. The WEPL value in cm or mm is used as input to find and compute the final dose. For a Monte Carlo dose engine the WEPL spread is not a problem as the path length spread in the three-dimensional object is accounted for automatically during the Monte Carlo calculation. Energy loss is calculated on the fly while the ion is traveling through the three-dimensional object.

The present disclosure further relates to a method for optimizing an existing intensity modulated particle therapy (IMPT) radiation treatment plan for a target in a patient, which target is represented by at least one voxel, preferably at least two voxels. The method comprises several steps that work together to enhance the optimization process.

In a first step, the method involves sampling a large number of ions directed towards the target, all having the same energy layer, from a radiation source.

Next the method involves placing a three-dimensional object, which has at least one spike measuring at least 2mm or at least 3mm up to 300mm in length, into the path of the sampled ions. The length of the spikes represents the water equivalent path length (WEPL), which may vary depending on the material used for the spikes. Each ion is then traced along a path through this three-dimensional object and the patient, until it reaches a voxel representing at least a part of the target. During this tracing process, a water equivalent path length (WEPL) value is calculated for each voxel and each ion. This WEPL value considers the varying densities of tissues the ion passes through until it reaches its Bragg peak. The calculated WEPL value for each ion is then inserted into a WEPL histogram per traversed voxel. This process of tracing ions and calculating WEPL values is repeated for a large number of ions and their respective traces for each voxel.

Once the WEPL histograms are populated with data for all the voxels representing the target, the next step involves calculating a dose for a single energy layer treatment plan. This is done using a pencil beam dose engine that, when computing dose for a particular voxel, loops over the WEPL histogram bins until all values in the histogram are accounted for.

This dose calculation process may be repeated for each voxel representing the target.

The method may further comprise the step of tracing each one of the plurality of ions along a ray/trace through the three-dimensional object along at least two straight line segments extending from a point within the at least one spike, in order to account for deflection by scattering of ions in material of the at least one spike.

To account for scattering in any of the spikes and a material of the spike, respectively, one or several scattering deflections by an angle sampled from a suitable distribution, may be applied. In the case of including one scattering event for a trace or ray that undeflected would traverse for example 3 cm of the spike, any point along those 3 cm in the spike may be selected and then a new direction may be sampled in order to then proceed with the raytracing using the new direction.

The three-dimensional object may comprise more than one spike, each with a length of at least 2mm. These spikes are oriented parallel to the path of the ions in order to affect the WEPL and therewith the range in the patient of the ion passing through the respective spike.

For computational efficiency and simplicity, the step of tracing each ion through the three-dimensional object may be done along a straight line.

Furthermore, the tracing of each ion and the calculation of the WEPL value can be performed from the voxel towards the radiation source or vice versa.

The method typically involves a large number of ions and traces, in the range of about 1 to 10 million ions and traces.

The method is applicable to various types of ions used in particle therapy, including heavier ions such as helium (He), lithium (Li), beryllium (Be), boron (B), carbon (C), nitrogen (N), oxygen (O), fluorine (F) or neon (Ne) ions.

In the context of this disclosure, the WEPL histogram is a WEPL distribution per voxel, providing a detailed radiological depth profile for each voxel.

The voxels used to represent the target are typically cubes in the millimeter range, providing a high-resolution representation of the target volume.

The voxel may be chosen to have a size of 2mm x 2mm x 2mm, thus in a cube range, but can also be chosen to be in a cube range from 1mm to 3mm or larger.

A potential application of this method is in the planning of FLASH radiation treatment, which involves the delivery of high doses of radiation in a very short time. The single energy treatment plan produced by this method may be particularly suitable for FLASH therapy.

In summary, the present disclosure provides a novel and improved method for optimizing IMPT radiation treatment plans. By incorporating a three-dimensional object with spikes into the ion tracing process, and by efficiently calculating WEPL values and dose distributions, this method enhances the accuracy and efficiency of the treatment planning process. This can lead to more precise and effective particle therapy treatments for cancer patients.

In this disclosure some expressions and terms are used, which are herewith briefly explained:

### Intensity Modulated Particle Therapy (IMPT)

Intensity Modulated Particle Therapy (IMPT) is a type of radiation treatment that uses a beam of ions, typically protons to irradiate diseased tissue, most often in the treatment of cancer. The beam of protons can be manipulated to deliver radiation to the tumor from several angles and at different intensities and energies and thus depths. This allows for a high dose of radiation to be delivered to the tumor, while minimizing the dose to the surrounding healthy tissue.

### Intensity modulated particle therapy (IMPT) radiation treatment plan

Herein the IMPT radiation treatment plan may basically be considered a starting point together with a pencil beam dose engine for the optimization method disclosed herein. The IMPT radiation treatment plan may use a pencil beam scanning method. It may also be an IMPT radiation treatment plan with a plurality energy layers.

### Voxel and dose voxel

A voxel is a unit of information that defines a point in three-dimensional space. Similar to a pixel which represents two-dimensional image data in a bitmap, a voxel represents volume data. In the context of this disclosure, a voxel or typically several voxels are used to represent a three-dimensional space. When calculating water equivalent path length voxels are used to simulate a path along which the ion travels from source to target. Such voxels can represent various material and tissue. Thus, such a voxel may represent a voxel in the three-dimensional object or it may represent a certain type of tissue in the patient. A dose voxel may be used to represent the target in the patient and the dose voxel is also used to calculate the actual dose. Herein the description relating to voxel and dose voxel can be applied to both the dose voxel and voxel, for example if the cube size of a voxel is described, such a cube size may also apply to the dose voxel and vice versa.

### Water Equivalent Path Length (WEPL)

Water Equivalent Path Length (WEPL) is a measure used in radiation therapy to determine the depth of penetration of an ion beam in tissue. It is defined as the length of a path in water that a proton, or any other charged particlem would need to travel to lose the same amount of energy as it would in the tissue.

### Three-Dimensional Object

In the context of this disclosure, a three-dimensional object is placed into the path of the ions in between the radiation source and the patient or target. This object has at least one spike measuring more than 3mm in length, typically about 3mm to 7mm in length. The ions are traced along a path through this object and the patient to a voxel, which is used to calculate the WEPL value. The three-dimensional object may be made of a polymer but can also be made of any other material. The shape of the spikes can be different from spike to spike, thus the three-dimensional object may comprise a variety of differently shaped spikes.

### Pencil Beam Dose Engine

A pencil beam dose engine is a type of dose calculation algorithm used in radiation therapy. It calculates the dose distribution in a patient by simulating the transport of an ion along a straight line and performing raytracing along the straight line while taking also lateral energy spread into account in an approximate manner.

### Energy Layer

An energy layer refers to a layer of radiation treatment that is delivered at a specific energy level. All ions in the same energy layer have their Bragg peaks located in a layer or plane in the patient. In radiation treatment devices the energy of the ion delivery can be changed, even though it is time consuming. An energy layer herein describes an energy level at which the ions can be delivered to the target. It is desirable to have a single energy layer for a treatment plan since changing the energy layer prolongs treatment time and therewith enhances discomfort and risk of reduced accuracy of the treatment due to potential patient movement.

### FLASH Radiation Treatment

FLASH radiation treatment is a novel form of radiotherapy that delivers high doses of radiation in ultra-fast treatment times, typically less than a second. This approach has shown promise in preclinical studies by sparing healthy tissue while effectively treating cancerous targets.

### Ray or ray trace

The term ray or ray trace refers to an ideal straight path along which an ion travels from source to target. Each ion has one ray or ray trace.

### Beam

A beam or radiation beam is a treatment beam comprising a large number of ions and therewith rays and ray traces. The beam may be used to treat a patient and may, in its cross section correspond, at least more or less, to the target in the patient. In radiotherapy treatment, radiation is normally provided from a number of different directions around the patient. Each of these directions are called a beam. Several beams are typically used to maximize the dose to the target while keeping the dose to surrounding tissue low. A part of dose planning is selecting appropriate beam directions.

### Bragg peak

As previously mentioned, in ion-based radiotherapy each ion will emit most of its energy towards the end of its path or ray, creating what is known as the Bragg peak. A goal in treatment planning is to ensure that the Bragg peaks of all beams and therewith ions are placed within the treatment volume or target, in such a way that all parts of the treatment volume receive the prescribed dose while minimizing dose to the surrounding volume and tissue. The position of the Bragg peak is affected by the kinetic energy Tp of each ion and by the properties of the tissue traversed by the ion. The values for Tp are selected so that the ions having the lowest energy will stop close to the nearest end of the treatment volume and the ions having the highest energy will stop close to the farthest end of the treatment volume, as seen from the radiation source. If there is a setup change, for example, if the patient moves so that the ion will traverse a different type of tissue, this will affect the position of the Bragg peak, which will in turn cause an deviation in the delivered dose.

### Pencil beam dose engine

A pencil beam dose engine is a computational algorithm used in radiation therapy dose planning (typically applied with ions such as protons), used to calculate dose in a voxel.

### Pencil beam scanning

Pencil beam scanning (PBS) is a delivery technique in radiation therapy (typically applied with ions for example protons), used to treat cancer by delivering a highly targeted beam of ions to a tumor. This pencil-thin beam, just a few millimeters wide, is steered magnetically to cover the tumor in a layer-by-layer fashion, ensuring a precise conformal dose distribution over the tumor.

Features of PBS include:
- The beam's small size, often less than 1 cm wide.
- The ability to rapidly change the beam's direction enabling a beam to cover a tumor volume in the body.
- The ability to change the beam's energy to control its depth, enabling the treatment of tumors at varying depths within the body.
- Spot scanning to deliver exact doses to specific tumor areas.

Advantages of PBS are:
- Enhanced precision in targeting the tumor, which helps protect surrounding healthy tissue.
- Greater protection of critical organs nearby the tumor site.

Generally, PBS is used with protons but herein the PBS is applied to other heavier ions such as helium (He), lithium (Li), beryllium (Be), boron (B), carbon (C), nitrogen (N), oxygen (O), fluorine (F) or neon (Ne) ions to find an optimized treatment plan.

### Monte Carlo method

The Monte Carlo (MC) method is an advanced computational technique used in, for example, radiation treatment calculations to simulate the behavior of radiation particles interacting with a medium, such as human tissue, offering high accuracy in dose distribution calculations. This statistical method uses random sampling to model the complex transport of radiation particles, crucial for radiotherapy dosimetry and treatment planning.

In a Monte Carlo dose calculation, the method enables precise calculations of radiation absorbed by the body ensuring that the treatment plan is designed so that a correct dose distribution in the patient's tumor and healthy tissue is achieved, minimizing dose to the healthy tissue. For treatment planning, MC simulations use 3D patient anatomy models from computer tomography (CT) data to evaluate how radiation moves through various structures, accommodating factors like tissue heterogeneity.

Renowned for its accuracy, the MC method can handle nearly all treatment scenarios, even with irregular tumor shapes and varied tissue types using protons. Yet, it is computationally demanding and can therewith not be used with heavier ions than protons.

### Monte Carlo dose engine

A Monte Carlo dose engine is a computational tool used in radiotherapy to calculate the dose distribution of radiation within a patient's body. This engine leverages the Monte Carlo method, which involves simulating the random paths of individual radiation particles and their interactions with matter to achieve highly accurate dose calculations. Monte Carlo dose engines represent a critical advancement in radiotherapy treatment planning, offering accuracy in dose calculations. Typically, a Monte Carlo dose engine only works sufficiently fast with lightweight particles such as photons, protons or electrons. By simulating the stochastic nature of radiation transport, these engines may improve precise delivery of therapeutic doses, thereby improving treatment outcomes and patient safety.

The difference between a Monte Carlo dose engine and a Monte Carlo method is that the Monte Carlo method is a statistical technique while a Monte Carlo dose engine is a specific application of this method in radiotherapy. The Monte Carlo method provides the theoretical foundation for simulating radiation transport and interactions, a Monte Carlo dose engine is a practical implementation of this method, which is typically integrated in radiation treatment planning systems to deliver accurate and efficient dose calculations in a clinical setting or environment.

### Brief Description of the Drawings

The disclosure will be described in more detail in the following, by way of example and with reference to the appended drawings, in which
- Figure 1:: schematically illustrates a set-up of a radiation treatment system with a resulting dose delivery;
- Figure 2:: schematically illustrates a three-dimensional object that can be inserted in rays of a beam of a radiation treatment;
- Figure 3:: schematically illustrates a set-up of a radiation treatment system with the three-dimensional object being inserted in a path of the rays of the beam with a resulting dose delivery;
- Figure 4:: schematically illustrates a similar set-up as figure 3 but instead of illustrating the dose delivery it shows how ions reach a target in a patient;
- Figure 5:: schematically illustrates an enlarged view of a part of figure 4 illustrating a detail of the target;
- Figure 6:: schematically illustrates a spike and a tracing of an ion tacking into account a scattering in the material of the spike;
- Figure 7:: schematically illustrates a method according to this disclosure; and
- Figure 8:: schematically illustrates a computer system enabled to perform the steps of method according to figure 6 in a radiation therapy system.

### Detailed description

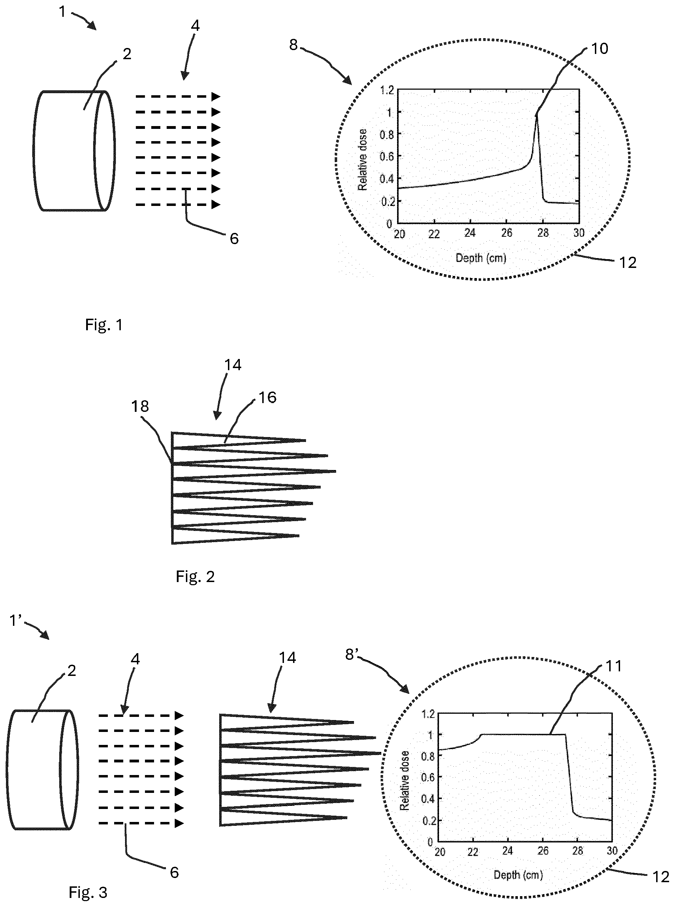

Figure 1 illustrates a radiation therapy system 1 having a radiation source 2 enabled to emit a beam 4 having several rays of ions 6 that results in a graph 8 of a radiation dose delivery. The rays of ions 6 travel from the radiation source 2 towards a patient 12 where it releases its energy along a depth within the patient 12 and a target (not illustrated in figure 1) respectively, which is illustrated in the graph 8. As can be seen from the relative dose vs the depth graph, a bragg peak 10 is formed towards the end of a ray of an ion. The bragg peak 10 is where the ion releases the highest energy amount of energy per traversed distance which happens towards the end of the ray just before the ion stops within the target. The graph 8 as shown in figure 1 illustrates the delivery of the relative dose for a single energy layer treatment plan, which basically means a fixed depth of the bragg peak 10.

The target may be a tumour or other cancer tissue.

It has been a goal to modify the dose delivery according to figure 1 to improve the dose distribution over the target. This may be achieved using a three-dimensional object 14, as shown in figure 2.

Figure 2 illustrates the three-dimensional object 14 having a plurality of spikes, pins or needles 16. The spikes 16 extend in the same direction but may be of different length. Their cross-sectional diameters are in the range of 0.2mm up to 5mm and their length may vary from 2mm to 300mm, depending on a shape of the target (not shown in figure 2). The spikes 16 are held together at their base 18 via base plate or the like. The spikes may have a cone shape, a pyramid shape or anything in between these two shapes. Alternatively, the spikes may be shaped as cylinders or pillars or as a rectangular solid. The three-dimensional object 14 may be made of a plastic, in particular plastic that can be 3D printed. In an embodiment the three-dimensional object may be made by 3D printing based on a radiation treatment plan that takes the shape of the target into account.

The spikes 16 of the three-dimensional object 14 may have different heights and shapes, selected in such a way that the beam 4 passing through the three-dimensional object 14 will be modulated to have Bragg peaks covering the whole of a target in a desired way. Of course, the size, shape and thickness of the three-dimensional object 14, the arrangement of the spikes on the three-dimensional object 14 and their size and heights, should be selected to conform to the target.

A three-dimensional object 14 as illustrated in figure 2 may then be used and inserted into a path of the beam 4 and therewith rays of the ions 6 in an improved radiation treatment planning system 1', as illustrated in figure 3. The insertion of such the three-dimensional object 14 then manipulates and changes the graph 8' and therewith the relative dose delivery 11 into a more homogenous distribution, which optimized dose within the target in the patient 12.

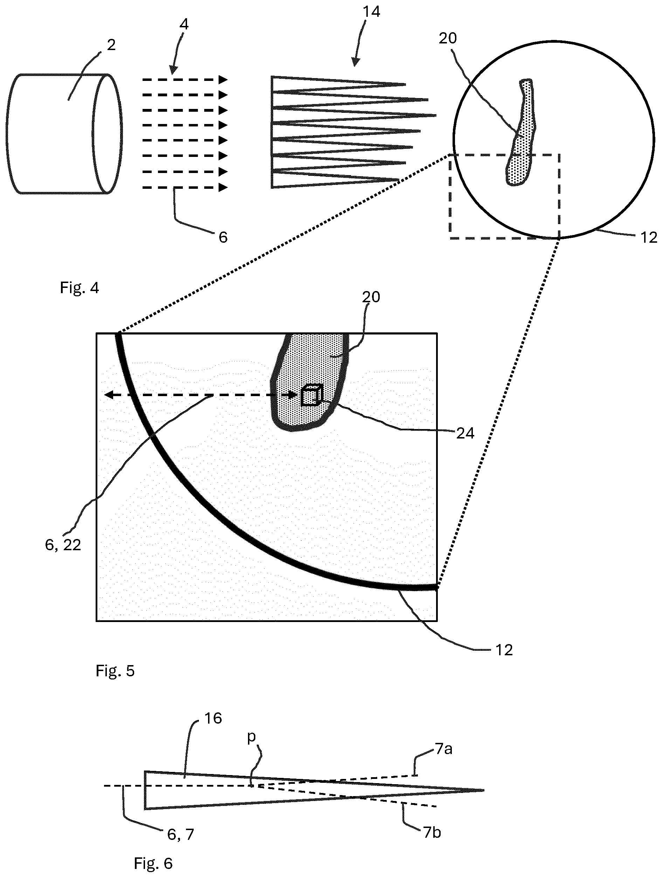

In an embodiment the three-dimensional object 14 may be 3D printed directly on the clinical site and used to treat a target 20 (c.f. figure 4). The three-dimensional object 14 is basically an inverted representation of the target 20 so that the radiation dose is optimally distributed in the target 20 during the radiation treatment.

Figure 4 illustrates a similar set up as figure 3 but this time the target 20 within the patient 12 instead of the graph of the dose distribution. As can be seen from the figure 4, the ions 6 of the beam 4 extend from the radiation source 2 and through three-dimensional object 14 into the target 20. The target 20 is modelled with voxels 24 as illustrated in figure 5, which is an enlargement of figure 4.

Figure 5 illustrates a path 22 of the ion 6 through the patient 12 to the voxel 24 that is representing a part of the target 20. The path 22 through patient 12 to the voxel 20 or target/dose voxel 20 can be modelled using voxels and then the actual dose distribution can be calculated based on water equivalent path lengths (WEPL). This means that the entire path 22 from the radiation source 2 to the target voxel 24 can be modelled or represented by voxels in order to calculate the dose distribution in the target 20. This may be done by modelling or representing the target 20 using a plurality of voxels 24 and by calculating the dose for each voxel representing the target 20. Each voxel representing the target 20 may be called target voxel or dose voxel 24. The path 22 may be called trace 22.

Figure 6 illustrates a single spike 16 of the three-dimensional object 14, as shown in figure 4, and a trace 7 into the spike 16. The step of tracing S05a each one of the plurality of ions 6 along a ray/trace 7 through a spike 16 of the three-dimensional object 14 accounts for scattering that may occur in the spike. To account for scattering a point p is chosen within the spike and two straight line segments 6a, 6b extending from the point p are then sampled to account for the scattering. There may be only one chosen line segment. This step S05a of choosing a point p within the spike 16 takes scattering into account.

Herein to account for scattering in any of the spikes 16 and a material of the spike, respectively, one or several scattering deflections by an angle sampled from a suitable distribution, may be applied. In the case of including one scattering event for a trace 7 or ray 7 that undeflected would traverse for example 3 cm of the spike 16, any point p along those 3 cm in the spike may be selected and then a new direction 7a, 7b may be sampled in order to then proceed with the raytracing using the new direction 7a, 7b.

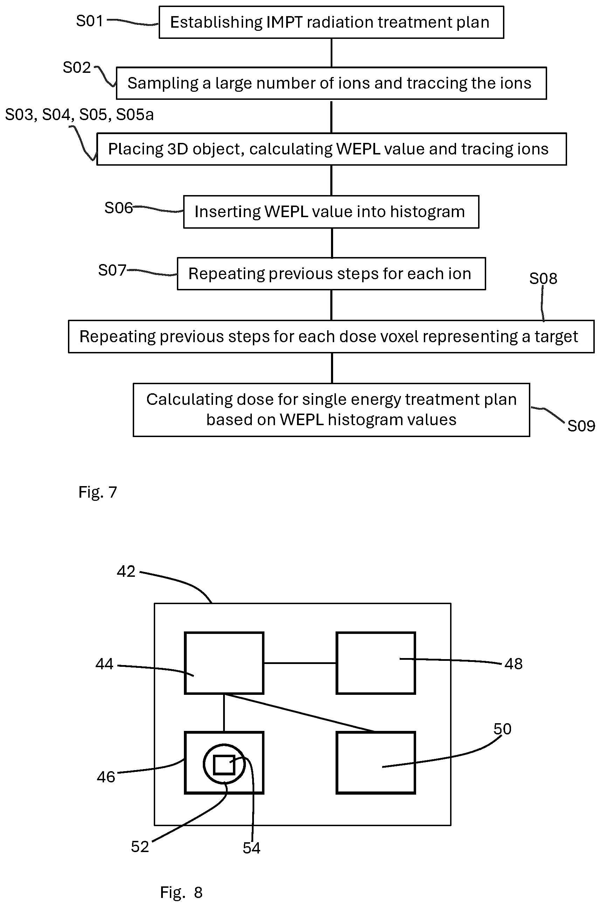

Figure 7 represents a method for optimizing an existing intensity modulated particle therapy, IMPT, radiation treatment plan for a target in a patient, which target is represented by at least two dose voxels, the method comprising the steps of:
- sampling S02 a large number of ions 6 for a dose voxel 16 all having a same energy layer from a radiation source 2, and
- placing S03 a three-dimensional object having at least one spike 18, measuring more than 3mm in length, into the path of the ions and tracing S04 each ion 6 along a trace 22 through the three-dimensional object 14 and the patient 12 to the dose voxel 24, thereby calculating S05 a water equivalent path length (WEPL) value for the dose voxel 24 and the ion 6 based on the IMPT radiation treatment plan.

Optionally the step of calculating S05 may further comprise the step S05a of tracing an ion through one of the spikes 16 and then change the angle of the ray 7 to account for scattering within the spike 16.

The method further comprises the steps of:
- inserting S06 the water equivalent path length value into a WEPL histogram, and
- repeating S07 the above steps S02 to S06 for the large number of ions 6 and therewith traces 22 for the dose voxel 24.

The previous steps S02 to S07 may then be repeated S08 for all dose voxels 24 representing the target 20 and then a dose for a single energy layer treatment plan using a pencil beam dose engine by looping over the WEPL histogram per dose voxel 24 is calculated S09 until all values in the WEPL histogram for the dose voxel(s) 24 are accounted for.

This may then be repeated for each dose voxel representing the target to arrive at a dose distribution over the entire target 20 and a single energy layer treatment plan using a pencil beam dose engine.

As a first initial step, an intensity modulated particle therapy, IMPT, radiation treatment plan for a target in a patient may be established S01. The generation S01 of such an IMPT is however an optional part of this disclosure.

Further, the three-dimensional object 12 may comprise more than one spike 16 with a length of at least 3mm.

The spikes 16 may be oriented parallel to the path 22 or trace 22 of the ions 6.

The step of tracing S04 each ion 6 along a trace 22 through the three-dimensional object 14 may be done along a straight line.

The step of tracing S04 each ion along a line through the three-dimensional object 14 and the patient 12 to the dose voxel 22, thereby calculating a water equivalent path length (WEPL) value for the voxel and the ion, may be performed from the dose voxel 22 towards a radiation source 2.

The large number of ions may be about 1 to 10 million ions and traces, 1 to 20 million ions and traces or 1 to 50 million ions and traces respectively.

The ions used herein for treatment may be any of helium (He), lithium (Li), beryllium (Be), boron (B), carbon (C), nitrogen (N), oxygen (O), fluorine (F) or neon (Ne) ions.

The WEPL histogram may be a WEPL distribution per dose voxel 22.

As shown and indicated in figure 5, a voxel is a cube in the millimeter range and whereby a plurality of dose voxels 22 are used to represent a target 20.

The single energy treatment plan may be for FLASH radiation treatment.

The least one spike 16 of the three-dimensional object has a diameter of 0.1mm or greater and whereby the three-dimensional object 14 covers at least a part of a beam 4 that extends from a source 2 to the target 20.

Figure 8 illustrates further a radiation therapy treatment planning system 42 comprising a processor 44, a memory 46 connected to the processor 44, an optional extra memory 50 also connected to the processor 44 and/or the memory 46 and an input/output interface 48 having a display and/or a keyboard for user interaction. The processor 44 may be any combination of a central processing unit (CPU), multiprocessor, digital signal processor (DSP), an ASIC (application specific integrated circuit) capable of executing software instructions stored on the memory and capable of executing the methods described according to the embodiments herein. The memory 46 and/or the extra memory 50 may comprise software code in the form of a computer program product 52 comprising computer software 54 or instructions that comprise any method according to this disclosure and that can perform any method according to this disclosure. The computer software 54 may be executed by the processor 44.

The memory 46 and the extra memory 50 may be any of a random access memory (RAM) or read only memory (ROM) or any combination thereof.

## Claims

1. A method for optimizing an existing intensity modulated particle therapy, IMPT, radiation treatment plan for a target (20) represented by at least two dose voxels (24), the method comprising the steps of:
- sampling a large number of ions (6) for the target (20) all having one energy layer from a radiation source (2);
- placing a three-dimensional object (14) having at least one spike (16), measuring at least 2 mm in length, into the path of the ions (6) and tracing each ion (6) along a trace (7) through the three-dimensional object (14) and the patient to one of the at least two dose voxels (24), thereby calculating a water equivalent path length (WEPL) value for the dose voxel and the ion (6) based on the IMPT radiation treatment plan;
- inserting the water equivalent path length value into a WEPL histogram;
- repeating the above steps for the large number of ions (6) and therewith traces for the at least two dose voxels. (24);
- calculating a dose distribution for a single energy layer treatment plan using a pencil beam dose engine by looping over the WEPL histogram per dose voxel until all values in the WEPL histogram are accounted for.

2. The method according to claim 1, wherein the three-dimensional object (14) comprises more than one spike (16) with a length of at least 2mm and wherein the spikes (16) are oriented parallel to the path (7) of the ions (6).

3. The method according to any of the preceding claims, wherein the step of tracing each ion (6) along a trace (7) through the three-dimensional object (14) is done along a straight line.

4. The method according to any of the preceding claims, wherein the step of tracing each ion (6) along a line through the three-dimensional object (14) and the patient to the dose voxel, thereby calculating a water equivalent path length (WEPL) value for the voxel and the ion (6), is performed from the dose voxel towards the radiation source (2).

5. The method according to any of the preceding claims wherein the large number of ions (6) is about 100 to 10 million ions and traces, respectively.

6. The method according to any of the preceding claims, wherein the ions are any of helium (He), lithium (Li), beryllium (Be), boron (B), carbon (C), nitrogen (N), oxygen (O), fluorine (F) or neon (Ne) ions.

7. The method according to any of the preceding claims, wherein the WEPL histogram is a WEPL distribution per dose voxel.

8. The method according to any of the preceding claims, wherein a voxel (24) is a cube in the millimeter range and wherein a plurality of dose voxels are used to represent the target (20).

9. The method according to any of the preceding claims, wherein the step of tracing each ion (6) along a trace (7) through the three-dimensional object (14) and the patient to one of the at least two dose voxels (24) is done along at least two straight line segments (7a, 7b) extending from a point (p) within the at least one spike (16), in order to account for deflection by scattering of ions (6) in material of the at least one spike (16).

10. The method according to any of the preceding claims, wherein the single energy treatment plan is used for FLASH radiation treatment.

11. The method according to any of the preceding claims, wherein the at least one spike (16) has a diameter of 0.1mm or greater and wherein the three-dimensional object (14) covers at least a part of a beam that extends from the radiation source (2) to the target (20).

12. A computer program product comprising computer readable means which, when executed in a computer, will cause the computer to perform the method according to any one of the preceding claims.

13. A radiation therapy planning system comprising a processor (44), a memory (46), wherein the memory (44) comprises a computer program product (52) according to the previous claim, the radiation therapy planning system being designed to perform the method according to any of the preceding claims 1 to 11.
